# EUROPEAN PATENT SPECIFICATION

(11) **EP 0 672 141 B1**
(45) Date of publication and mention of the grant of the patent: **14.05.2003**
(21) Application number: 94900364.4
(22) Date of filing: 20.10.1993
(51) Int. Cl.: C12N 15/12, C12N 15/62

(54) **METHODS OF PREPARING SOLUBLE, OLIGOMERIC PROTEINS**
METHODEN ZUR HERSTELLUNG LÖSLICHER, OLIGOMERER PROTEINE
PROCEDE DE PREPARATION DE PROTEINES OLIGOMERES SOLUBLES

(30) Priority: 23.10.1992 US 969703; 13.08.1993 US 107353
(43) Date of publication of application: 20.09.1995
(73) Proprietor: IMMUNEX CORPORATION, Seattle Washington 98101 (US)
(72) Inventor: SPRIGGS, Melanie, K., Seattle, WA 98119 (US); SRINIVASAN, Subhashini, Kirkland, WA 98034 (US)
(74) Representative: Cornish, Kristina Victoria Joy
(86) International application number: US9310034
(87) International publication number: WO94010308

(56) References cited:
- EP-A- 0 325 224
- EP-A- 0 413 622
- US-A- 5 073 627
- BLONDEL A. AND BEDOUELLE H.: "Engineering the quaternary structure of an exported protein with a leucine zipper" PROTEIN ENGINEERING, vol. 4, no. 4, April 1991, pages 457-461, XP002044838
- HODGES R.: "Unzipping the secrets of coiled-coil" CURRENT BIOLOGY, vol. 2, no. 3, March 1992, pages 122-124, XP002044839
- The Journal of Immunology, Vol. 148, No. 5, issued 01 March 1992, KOSTENLY et al., "Formation of a Bispecific Antibody by the Use of Leucine Zippers", pages 1547 to 1553, see entire document.
- KOSTENLY ET AL: 'Formation of a bispecific antibody by the use of Leucine Zippers' THE JOURNAL OF IMMUNOLOGY vol. 148, no. 5, pages 1547 - 1553

## Description

### TECHNICAL FIELD OF THE INVENTION

The present invention relates to a method of preparing soluble oligomeric proteins using recombinant DNA technology.

### BACKGROUND OF THE INVENTION

The biological activity of proteins is dependent upon proper tertiary and quaternary structure, or conformation. Many proteins exist as oligomers (structures comprised of two or more polypeptide chains) in their native form. Such oligomers are often stabilized by non-covalent interactions, and are thus dependent on proper tertiary structure of the individual peptides. Expression of a recombinant protein in biologically active form, exhibiting the proper tertiary and quaternary structure, by host cells which do not normally express a native form of the protein, frequently presents a significant challenge. Of particular interest in recombinant protein technology is expression of proteins that are membrane-bound in the biologically active form, as soluble proteins. Soluble proteins are useful as therapeutic agents, and in other applications requiring large quantities of highly purified proteins.

Soluble forms of transmembrane proteins have been prepared by deleting the transmembrane and intracytoplasmic domains, and adding an appropriate signal peptide to enable secretion of the soluble form of the protein (Smith et al., *Science* 238:1704, 1987; Treiger et al., *J. Immunol.* 136:4099, 1986). Some soluble proteins have been expressed as fusion proteins in which the extracellular domain of the membrane protein is joined to an immunoglobulin heavy chain constant region (Fanslow et al., J. *Immunol*. 149:65, 1992; Noelle et al., *Proc. Natl. Acad. Sci. U.S.A.* 89:6550, 1992), or with the extracellular domain of the murine T lymphocyte antigen CD8 (Hollenbaugh et al., *EMBO J*. 11:4313, 1992). However, such soluble proteins may not be biologically active due to improper tertiary and/or quaternary structure. Some soluble forms of transmembrane proteins may be biologically active, but poorly expressed, or unstable under the conditions of expression or purification, due to changes in structure as a result of deletion of a portion or portions of the protein. EP-A-0325224 discloses methods for producing secreted receptor analogs such as PDGF-R analogs, and methods for preparing biologically active dimers by fusing a dimerizing protein such as the immunoglobulin heavy chain region to a peptide that requires dimerization for activity.

Leucine zipper is a term that is used to refer to. a repetitive heptad motif containing four to five leucine residues present as a conserved domain in several proteins. Leucine zippers fold as short, parallel coiled coils, and are believed to be responsible for oligomerization of the proteins of which they form a domain. Sequences derived from the *fos* and *jun* leucine zippers have been used in the formation of bispecific antibodies by expression of DNA encoding the V_{L} and V_{H} regions of antibodies as fusion proteins with the leucine zipper sequences. (Kostelny et al., *J. Immunol*. 148:1547, 1992) Leucine zipper sequences have also been used to replace the dimerization domain of λ repressor, a soluble DNA-binding protein of bacteriophage λ (Hu et al., *Science* 250:1400, 1990), and in the preparation of a dimeric form of MalE, a maltose binding protein of *E. coli* that is exported into the periplasmic space (Blondel and Bedoulle, *Protein Engineering* 4:457, 1991).

There is a need in the art to develop methods of expressing biologically active, recombinant, oligomeric proteins, particularly soluble proteins that are membrane-bound in their biologically active configuration.

### SUMMARY OF THE INVENTION

The present invention relates to a method of preparing a soluble, oligomeric mammalian protein by culturing a host cell transformed or transfected with an expression vector encoding a fusion protein comprising a leucine zipper domain and a heterologous mammalian protein. In one embodiment, the heterologous mammalian protein comprises an extracellular domain of a mammalian transmembrane protein; the resulting fusion protein forms an oligomer. In another embodiment, the heterologous mammalian protein comprises a soluble protein such as a cytokine; the resulting fusion protein forms an oligomer. In another embodiment, the leucine zipper domain is removed from the fusion protein, by cleavage with a specific proteolytic enzyme. In another embodiment, a hetero-oligomeric protein is prepared by utlizing leucine zipper domains that preferentially form hetero-oligomers.

### BRIEF DESCRIPTION OF THE DRAWINGS

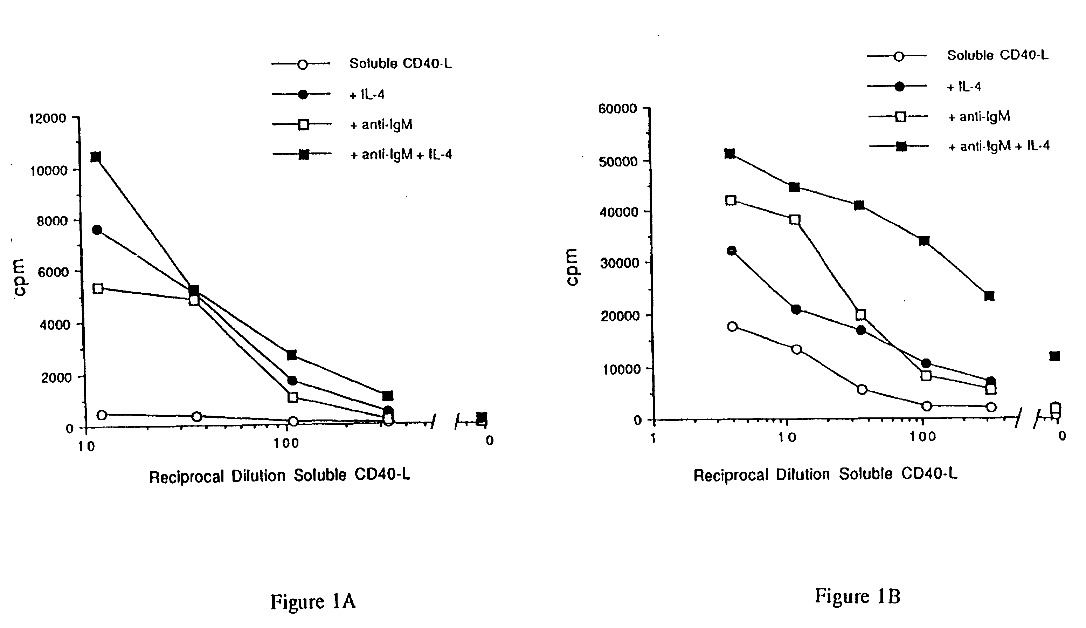

Figure 1A illustrates the ability of soluble, oligomeric human CD40-L comprising a leucine zipper domain to stimulate the proliferation of human tonsillar B cells; Figure 1B illustrates the ability of soluble, oligomeric human CD40-L comprising a leucine zipper domain to stimulate the proliferation of human peripheral blood B cells.

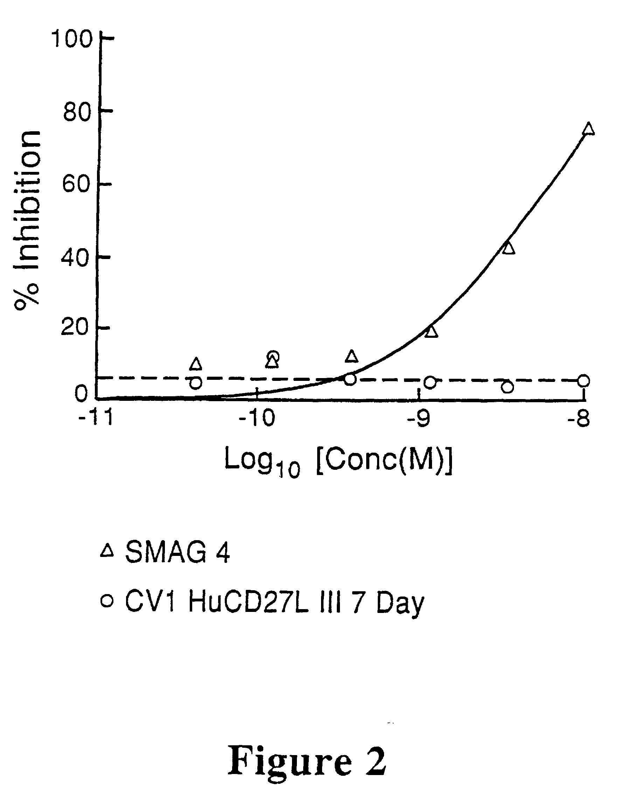

Figure 2 illustrates the inhibition of binding of CD27.Fc to MP.1 cells, which express CD27-L, by a soluble form of CD27-L, sCD27L-3, that comprises a leucine zipper domain. Curve 1 (---): I=6.55; K=7.84e+11; Kₐ=2.93e+07; MF=8.84e-10. Curve 2 (―): I=111.49; K=2.21e+08; Kₐ=2.93e+07; MF=8.83e-10

### DETAILED DESCRIPTION OF THE INVENTION

The present invention relates to a method as set out in any one of claims 1-10.

Exemplary mammalian transmembrane proteins include members of the tumor necrosis factor/nerve growth factor receptor (TNFR/NGFR) family (Farrah and Smith, *Nature* 358:26, 1992; Goodwin et al., *Cell* 73:447; 1993), which includes CD40 Ligand (CD40-L), CD27. Ligand (CD27-L), OX40 Ligand (OX40-L), and TNF. Structural studies of certain members of this family of proteins indicate that they form homotrimers. The inventive method will also be useful for other members of this family.

Additionally, many other mammalian transmembrane proteins form oligomers, either hetero-oligomers or homo-oligomers, in their biologically-active form. Members of the hematopoietin receptor family (Cosman et al., *Trends Biochem. Sci*. 15:265; 1990) are exemplary of such proteins. Gearing et al. *(Science* 255:1434, 1992) reported the cloning of a gene encoding a protein (gp130) that conferred high-affinity binding to both leukemia-inhibitory factor (LIF) and Oncostatin M (OSM) when expressed in cells along with a low-affinity LIF receptor. Similar interactions of a low-affinity receptor and a second subunit protein, resulting in a high-affinity receptor have also been proposed for other members of this family (Hayashida et al., *Proc. Natl. Acad. Sci. U.S.A*. 87:0655, 1990; Kitamura et al., *Cell* 66:1165, 1991; Tavernier et al., *Cell* 66:1175, 1991; Devos et al., *EMBO J*. 10:2133, 1991). Soluble forms of the members of the hematopoietin receptor family will exhibit higher affinity for their cognate ligand when expressed as hetero-oliogmers, or in some cases, as homo-oligomers. The same will be true for other transmembrane proteins that comprise two or more subunits.

In another embodiment, the heterologous mammalian protein comprises a soluble protein such as a cytokine; the resulting fusion protein forms an oligomer. Cytokines are soluble mediators released by cells during an immune or inflammatory response, which provide antigenically non-specific, intracellular signals that are crucial in regulating physiological processes. TNF α, TNF β and certain neurotrophins such as nerve growth factor (NGF) belong to the TNF/NGF family. Modeling studies of certain members of this family indicate that they are likely to form oligomers (Goh and Porter, *Protein Eng.* 4:385, 1991; Peitsch and Jongeneel, *Int. Immunol*. 5:233, 1993). Furthermore, other cytokines, including macrophage colony stimulating factor (M-CSF; Pandit et al., *Science* 258:1358, 1992) are also known to be oligomeric. Such cytokines will also be useful in the inventive method, wherein a leucine zipper domain stabilizes the proper quaternary structure of the oligomeric cytokine.

In another embodiment, hetero-oligomeric forms of cytokines are prepared. A fusion protein of granulocyte-macrophage colony stimulating factor (GM-CSF) and Interleukin-3 (IL-3) has been shown to be a more potent proliferation stimulus than either factor alone or IL-3 and GM-CSF combined (U.S. Patents 5,073,627 and 5,108,910). Fusion proteins comprising GM-CSF and IL-3 and DNA sequences encoding such fusion proteins are described in U.S. Patents 5,073,627 and 5,108,910, respectively, both of which are incorporated by reference herein. A similar, bivalent protein composed of GM-CSF and IL3 may be formed by the expression of these cytokines as fusion proteins comprising leucine zipper domains that preferentially form heterodimers.

In another embodiment, the leucine zipper domain is removed from the fusion protein, for example by cleavage with a specific proteolytic enzyme. In addition to a leucine zipper sequence and a heterologous protein, such fusion proteins also comprise an amino acid sequence recognized, and cleaved, by a selected proteolytic enzyme. The leucine zipper domain functions to stabilize the recombinant fusion protein during expression and secretion. After purification of the secreted protein, the leucine zipper is enzymatically removed by treating with the proteolytic enzyme. The heterologous protein may then become monomeric. Such monomeric forms of soluble proteins will be useful as receptor antagonists, for example, by binding to a cognate receptor and preventing signaling by preventing cross-linking of the receptor.

### Leucine zipper domains

Leucine zippers were originally identified in several DNA-binding proteins (Landschulz et al., *Science* 240:1759, 1988). Leucine zipper domain is a term used to refer to a conserved peptide domain present in these (and other) proteins, which is responsible for dimerization of the proteins. The leucine zipper domain (also referred to herein as an oligomerizing, or oligomer-forming, domain) comprises a repetitive heptad repeat, with four or five leucine residues interspersed with other amino acids. Examples of leucine zipper domains are those found in the yeast transcription factor GCN4 and a heat-stable DNA-binding protein found in rat liver (C/EBP; Landschulz et al., *Science* 243:1681, 1989). Two nuclear transforming proteins, *fos* and *jun*, also exhibit leucine zipper domains, as does the gene product of the murine proto-oncogene, *c-myc* (Landschulz et al., *Science* 240:1759, 1988). The products of the nuclear oncogenes *fos* and *jun* comprise leucine zipper domains preferentially form a heterodimer (O'Shea et al., *Science* 245:646, 1989; Turner and Tjian, *Science* 243:1689, 1989). The leucine zipper domain is necessary for biological activity (DNA binding) in these proteins.

The fusogenic proteins of several different viruses, including paramyxovirus, coronavirus, measles virus and many retroviruses, also possess leucine zipper domains (Buckland and Wild, *Nature* 338:547,1989; Britton, *Nature* 353:394, 1991; Delwart and Mosialos. *AIDS Research and Human Retroviruses* 6:703, 1990), The leucine zipper domains in these fusogenic viral proteins are near the transmembrane region of the proteins; it has been suggested that the leucine zipper domains could contribute to the oligomeric structure of the fusogenic proteins. Oligomerization of fusogenic viral proteins is involved in fusion pore formation (Spruce et al, *Proc. Natl. Acad. Sci. U.S.A*. 88:3523, 1991). Leucine zipper domains have also been recently reported to play a role in oligomerization of heat-shock transcription factors (Rabindran et al., *Science 259:230,* 1993).

Leucine zipper domains fold as short, parallel coiled coils. (O'Shea et al., *Science* 254:539; 1991) The general architecture of the parallel coiled coil has been well characterized, with a "knobs-into-holes" packing as proposed by Crick in 1953 (*Acta Crystallogr.* 6:689). The dimer formed by a leucine zipper domain is stabilized by the heptad repeat, designated (*abcdefg*)ₙ according to the notation of McLachlan and Stewart (*J. Mol. Biol.* 98:293; 1975), in which residues *a* and *d* are generally hydrophobic residues, with *d* being a leucine, which line up on the same face of a helix. Oppositely-charged residues commonly occur at positions *g* and *e*. Thus, in a parallel coiled coil formed from two helical leucine zipper domains, the "knobs" formed by the hydrophobic side chains of the first helix are packed into the "holes" formed between the side chains of the second helix.

The leucine residues at position *d* contribute large hydrophobic stabilization energies, and are important for dimer formation (Krystek et al., *Int. J. Peptide Res.* 38:229, 1991). Lovejoy et al. recently reported the synthesis of a triple-stranded α-helical bundle in which the helices run up-up-down *(Science* 259:1288, 1993). Their studies confirmed that hydrophobic stabilization energy provides the main driving force for the formation of coiled coils from helical monomers. These studies also indicate that electrostatic interactions contribute to the stoichiometry and geometry of coiled coils.

Several studies have indicated that conservative amino acids may be substituted for individual leucine residues with minimal decrease in the ability to dimerize; multiple changes, however, usually result in loss of this ability (Landschulz et al., *Science* 243:1681, 1989; Turner and Tjian, *Science* 243:1689, 1989; Hu et al., *Science* 250:1400, 1990). van Heekeren et al. reported that a number of different amino residues can be substituted for the leucine residues in the leucine zipper domain of GCN4, and further found that some GCN4 proteins containing two leucine substitutions were weakly active (*Nucl. Acids Res.* 20:3721, 1992). Mutation of the first and second heptadic leucines of the leucine zipper domain of the measles virus fusion protein (MVF) did not affect syncytium formation (a measure of virally-induced cell fusion): however, mutation of all four leucine residues prevented fusion completely (Buckland et al., *J. Gen. Virol.* 73:1703, 1992). None of the mutations affected the ability of MVF to form a tetramer.

Recently, amino acid substitutions in the *a* and *d* residues of a synthetic peptide representing the GCN4 leucine zipper domain have been found to change the oligomerization properties of the leucine zipper domain (Alber, Sixth Symposium of the Protein Society, San Diego, CA). When all residues at position *a* are changed to isoleucine, the leucine zipper still forms a parallel dimer. When, in addition to this change, all leucine residues at position *d* are also changed to isoleucine, the resultant peptide spontaneously forms a trimeric parallel coiled coil in solution. Substituting all amino acids at position *d* with isoleucine and at position *a* with leucine results in a peptide that tetramerizes. Peptides containing these substitutions are still referred to as leucine zipper domains since the mechanism of oligomer formation is believed to be the same as that for traditional leucine zipper domains such as those described above. However, prior to the present invention, the effect of these substitutions upon longer peptides of which the leucine zipper is but a small domain was not known, nor was it known if peptides comprising these sequences could be expressed and secreted by cells.

### Preparation of Gene Fragments and Oligonucleotides

Oligonucleotide fragments of about 12 to about 20 nucleotides may be prepared according to methods that are known in the art, for example, by using an automated DNA synthesizer. Several such fragments may be synthesized, which encode overlapping portions of a peptide, for example, a leucine zipper domain. Due to the degeneracy of the genetic code, most amino acids are encoded by two or more different nucleotide triplets. The selection of a triplet to encode a given amino acid will depend upon the organism in which the final gene product is to be expressed, among other considerations. Overlapping fragments may then be joined to form a DNA encoding a peptide of interest.

A polymerase chain reaction (PCR) technique (Saiki et al., *Science* 239:487, 1988) may be employed to amplify gene fragments encoding all or a portion of a protein of interest, using 5' (upstream) and 3' (downstream) oligonucleotide primers derived from the known DNA sequence of the gene, or a gene encoding a related protein. An exemplary set of PCR conditions includes: one cycle at 94°C for 2 minutes, followed by 42°C for two minutes; 30 cycles at 72°C for 1.5 minutes, followed by 94°C for one minute, then 48°C for 1 minute; and one cycle at 72°C for seven minutes. Restriction enzyme sites can also be added to the DNA sequences of interest, in order to facilitate ligation of the resulting PCR product with a plasmid or vector, or with an additional DNA sequence or sequences. Amplified DNA sequences may be joined substantially as described by Yon and Fried *(Nucleic Acids Res.* 17:4895; 1989).

For example, as disclosed in U.S.S.N. 08/097,827, filed July 23, 1993, the disclosure of which is incorporated by reference herein, full length mouse OX40 was cloned using 5' (upstream) and 3' (downstream) oligonucleotide primers based on the published sequence of rat OX40. The upstream primer comprised a recognition site for the restriction endonuclease *Spe* 1 upstream of a sequence encoding the first six (N-terminal) amino acids of rat OX40. The downstream primer comprised a recognition site for the restriction endonuclease *Spe* I upstream of a sequence encoding the last five (C-terminal) amino acids of full-length OX40. The PCR product was digested with *Spe I,* and an approximately 800 bp fragment was isolated by gel filtration, and used in a second round of PCR reaction. The isolated fragment was ligated into *Spe* I cut plasmid, pBLUESCRIPT SK® (Stratagene Cloning Systems, La Jolla, CA), which had been treated with calf intestine alkaline phosphatase (CIAP) to prevent self-ligation.

In another example, a DNA encoding only the extracellular region of a transmembrane protein can be obtained by deleting DNA encoding the intracellular and transmembrane portions of the transmembrane protein. Methods to determine which residues should be deleted and for performing the actual deletions are well known in the art. For example, Smith et al. describe a soluble form of the human CD4 antigen prepared by deleting the transmembrane and intracellular portions of the CD4 antigen *(Science* 238:1704, 1987). Treiger et al. prepared a soluble form of an Interleukin-2 receptor using similar methods using similar methods (*J. Immunol.* 136:4099, 1986).

A fusion protein may be formed from an extracellular region and a protein (or portion thereof) that is known to be secreted. For example, soluble proteins comprising an extracellular domain from a membrane-bound protein and an immunoglobulin heavy chain constant region was described by Fanslow et al., *J. Immunol*. 149:65, 1992 and by Noelle et al., *Proc. Natl. Acad. Sci. U.S.A.* 89:6550, 1992. The extracellular domain of the murine T lymphocyte antigen CD8 has also be utilized to form soluble fusion proteins (Hollenbaugh et al., *EMBO J.* 11:4313, 1992).

### Preparation of Fusion Proteins

Fusion proteins are polypeptides that comprise two or more regions derived from different, or heterologous, proteins or peptides. Fusion proteins are prepared using conventional techniques of enzyme cutting and ligation of fragments from desired sequences. PCR techniques employing synthetic oligonucleotides may be used to prepare and/or amplify the desired fragments. Overlapping synthetic oligonucleotides representing the desired sequences can also be used to prepare DNA constructs encoding fusion proteins. Fusion proteins can comprise several sequences, including a leader (or signal peptide) sequence, linker sequence, a leucine zipper sequence, or other oligomer-forming sequences, and sequences encoding highly antigenic moieties that provide a means for facile purification or rapid detection of a fusion protein.

Signal peptides facilitate secretion of proteins from cells. An exemplary signal peptide is the amino terminal 25 amino acids of the leader sequence of murine interleukin-7 (IL-7: Namen et al., *Nature* 333:571; 1988). Other signal peptides may also be employed furthermore, certain nucleotides in the IL-7 leader sequence can be altered without altering the amino acid sequence. Additionally, amino acid changes that do not affect the ability of the IL-7 sequence to act as a leader sequence can be made.

The Flag® octapeptide (SEQ ID NO:1; Hopp et al., *Bio*/*Technology* 6:1204, 1988) does not alter the biological activity of fusion proteins, is highly antigenic and provides an epitope reversibly bound by a specific monoclonal antibody, enabling rapid detection and facile purification of the expressed fusion protein. The Flag® sequence is also specifically cleaved by bovine mucosal enterokinase at the residue immediately following the Asp-Lys pairing. Fusion proteins capped with this peptide may also be resistant to intracellular degradation in *E. coli.* A murine monoclonal antibody that binds the Flag® sequence has been deposited with the ATCC under accession number HB 9259; methods of using the antibody in purification of fusion proteins comprising the Flag® sequence are described in U.S. Patent 5,011,912, which is incorporated by reference herein.

A protein of interest may be linked directly to another protein to form a fusion protein; alternatively, the proteins may be separated by a distance sufficient to ensure that the proteins form proper secondary and tertiary structures. Suitable linker sequences (1) will adopt a flexible extended conformation. (2) will not exhibit a propensity for developing an ordered secondary structure which could interact with the functional domains of fusion proteins, and (3) will have minimal hydrophobic or charged character which could promote interaction with the functional protein domains. Typical surface amino acids in flexible protein regions include Gly, Asn and Ser. Virtually any permutation of amino acid sequences containing Gly, Asn and Ser would be expected to satisfy the above criteria for a linker sequence. Other near neutral amino acids, such as Thr and Ala, may also be used in the linker sequence. The length of the linker sequence may vary without significantly affecting the biological activity of the fusion protein. Linker sequences are unnecessary where the proteins being fused have non-essential N- or C-terminal amino acid regions which can be used to separate the functional domains and prevent steric interference. Exemplary linker sequences are described in U.S. patents 5,073,627 and 5,108,910, the disclosures of which are incorporated by reference herein.

When an oligomeric fusion protein is formed from the extracellular portion of a transmembrane protein, a DNA sequence encoding an oligomer-forming domain, such as a leucine zipper domain, is fused to a DNA sequence encoding the extracellular region of the transmembrane protein. The members of the fusion protein are joined such that the oligomer-forming domain of the fusion protein is located in the same orientation relative to the fusion protein as the transmembrane and intracytoplasmic regions of the native transmembrane protein. An oligomeric fusion protein will be stabilized by the coiled-coil interaction of leucine zipper domain. Thus, in one example, a fusion protein comprising an extracellular region derived from a ligand for CD40 (CD40-L), a type II transmembrane protein described in U.S.S.N. 07/969,703, the disclosure of which is incorporated by reference herein, the oligomer-forming domain, a leucine zipper sequence, is fused to the amino-proximal end of the extracellular region. In a fusion protein derived from a type I transmembrane protein. the oligomer-forming domain would be fused to the carboxy-proximal end of the extracellular region of the type I transmembrane protein. Other transmembrane proteins traverse the cell membrane more than once. Such transmembrane proteins will have two or more different extracellular regions. Soluble, oligomeric fusion proteins may also be prepared from two or more of such different extracellular regions from the same transmembrane protein.

Oligomeric forms of proteins that occur naturally in soluble form may also be prepared. In such cases, the oligomer-forming domain is joined to the soluble protein such that formation of an oligomer follows the conformation of the biologically active, soluble protein. Furthermore, either homo-oligomeric proteins or hetero-oligomeric proteins can be prepared, depending upon the whether the oligomerizing domain(s) of the fusion protein preferentially form hetero-oligomers or homo-oligomers.

### Expression Vectors

Recombinant expression vectors for expression of a fusion protein comprising an oligomer-forming domain and a heterologous mammalian protein by recombinant DNA techniques include a DNA sequence comprising a synthetic or cDNA-derived DNA fragment encoding an oligomer-forming domain, linked in frame to a DNA fragment encoding the heterologous protein. These DNA fragments are operably linked to suitable transcription and/or translational regulatory nucleotide sequences, such as those derived from a mammalian, microbial, viral, or insect gene. Examples of regulatory sequences include sequences having a regulatory role in gene expression (e.g., a transcription promoter or enhancer), an operator sequence to control transcription, a sequence encoding an mRNA ribosomal binding site, a polyadenylation site, splice donor and acceptor sites, and appropriate sequences which control transcription, translation initiation and termination. In addition, sequences encoding signal peptides can be incorporated into expression vectors. For example, a DNA sequence for a signal peptide (secretory leader) may be operably linked to a DNA encoding a fusion protein comprising an oligomer-forming domain and a heterologous mammalian protein. The signal peptide is expressed as a part of a precursor amino acid sequence; the signal peptide enables improved extracellular secretion of translated fusion polypeptide by a yeast host cell.

Nucleotide sequences are operably linked when the regulatory sequence functionally relates to the DNA encoding the fusion protein. Thus, a promoter nucleotide sequence is operably linked to a DNA encoding a fusion protein if the promoter nucleotide sequence controls the transcription of the DNA encoding the fusion protein. Still further, a ribosome binding site may be operably linked to a sequence for a fusion protein if the ribosome binding site is positioned within the vector to encourage translation.

Transcription and translational control sequences for mammalian host cell expression vectors may be excised from viral genomes. For example, commonly used mammalian cell promoter sequences and enhancer sequences are derived from Polyoma virus, Adenovirus 2, Simian Virus 40 (SV40), and human cytomegalovirus. DNA sequences derived from the SV40 viral genome, for example, the SV40 origin, early and late promoters, enhancer, splice, and polyadenylation sites may be used to provide the other genetic elements required for expression of a structural gene sequence in a mammalian host cell. Viral early and late promoters are particularly useful because both are easily obtained from a viral genome as a fragment which may also contain a viral origin of replication (Fiers et al., *Nature 273*:113, 1978). Smaller or larger SV40 fragments may also be used, provided the approximately 250 bp sequence extending from the *Hind* III site toward the *Bgl* I site located in the SV40 viral origin of replication site is included.

Exemplary mammalian expression vectors can be constructed as disclosed by Okayama and Berg (*Mol. Cell. Biol. 3*:280, 1983). A useful high expression vector, PMLSV N1/N4, described by Cosman et al., *Nature 312*:768, 1984 has been deposited as ATCC 39890. Additional useful mammalian expression vectors are described in EP-A-0367566, and in U.S. Patent Application Serial No. 07/701,415, filed May 16, 1991, incorporated by reference herein. For expression of a type II protein extracellular region, such as OX40-L, a heterologous signal sequence may be added, such as the signal sequence for interleukin-7 (IL-7) described in United States Patent 4,965,195, or the signal sequence for interleukin-2 receptor described in United States Patent Application 06/626,667 filed on July 2, 1984. Another exemplary vector is pDC406, which includes regulatory sequences derived from SV40, human immunodeficiency virus (HIV), and Epstein-Barr virus (EBV).

Expression vectors transfected into prokaryotic host cells generally comprise one or more phenotypic selectable markers. A phenotypic selectable marker is, for example, a gene encoding a protein that confers antibiotic resistance or that supplies an autotrophic requirement, and an origin of replication recognized by the host to ensure amplification within the host. Other useful expression vectors for prokaryotic host cells include a selectable marker of bacterial origin derived from commercially available plasmids. This selectable marker can comprise genetic elements of the cloning vector pBR322 (ATCC 37017). pBR322 contains genes for ampicillin and tetracycline resistance and thus provides simple means for identifying transformed cells. The pBR322 "backbone" sections are combined with an appropriate promoter and a OX40-L DNA sequence. Other commercially vectors include, for example, pKK223-3 (Pharmacia Fine Chemicals, Uppsala, Sweden) and pGEM1 (Promega Biotec, Madison, WI, USA).

Promoter sequences are commonly used for recombinant prokaryotic host cell expression vectors. Common promoter sequences include β-lactamase (penicillinase), lactose promoter system (Chang et al., *Nature 275*:615, 1978; and Goeddel et al., *Nature 281*:544, 1979), tryptophan (trp) promoter system (Goeddel et al., *Nucl. Acids Res. 8:4057,* 1980; and EP-A-36776) and tac promoter (Maniatis, *Molecular Cloning: A Laboratory Manual*, Cold Spring Harbor Laboratory, p. 412, 1982). A particularly useful prokaryotic host cell expression system employs a phage λ P_{L} promoter and a cI857ts thermolabile repressor sequence. Plasmid vectors available from the American Type Culture Collection which incorporate derivatives of the λ P_{L} promoter include plasmid pHUB2 (resident in *E. coli* strain JMB9 (ATCC 37092)) and pPLc28 (resident in *E. coli* RR1 (ATCC 53082)).

### Host Cells

Suitable host cells for expression of a fusion protein comprising an oligomer-forming domain and a heterologous mammalian protein include prokaryotes and yeast or higher eukaryotic cells. Prokaryotes include gram negative or gram positive organisms, for example, *E. coil* or *Bacilli*. Suitable prokaryotic host cells for transformation include, for example, *E. coli, Bacillus subtilis, Salmonella typhimurium*, and various other species within the genera *Pseudomonas, Streptomyces,* and *Staphylococcus*. Higher eukaryotic cells include established cell lines of mammalian origin. Cell-free translation systems could also be employed to produce a fusion protein comprising an oligomer-forming domain and a heterologous mammalian protein using an RNA derived from DNA constructs disclosed herein.

In a prokaryotic host cell, such as *E. coli,* a fusion protein may include an N-terminal methionine residue to facilitate expression of the recombinant polypeptide in the prokaryotic host cell. The N-terminal Met may be cleaved from the expressed recombinant fusion protein. Prokaryotic host cells may be used for expression of fusion proteins that do not require extensive proteolytic or disulfide processing.

Appropriate cloning and expression vectors for use with bacterial, fungal, yeast, and mammalian cellular hosts are described, for example, in Pouwels et al. *Cloning Vectors: A Laboratory Manual,* Elsevier, New York, (1985). An expression vector carrying the recombinant fusion protein DNA is transfected or transformed into a substantially homogeneous culture of a suitable host microorganism or mammalian cell line according to methods that are known in the art, to form transfected or transformed host cells that express the fusion protein. Expressed fusion protein will be located within the host cell and/or secreted into culture supernatant fluid, depending upon the nature of the host cell and the gene construct inserted into the host cell.

A fusion protein comprising an oligomer-forming domain and a heterologous mammalian protein may be expressed in yeast host cells, preferably from the *Saccharomyces* genus (e.g., *S. cerevisiae*). Other genera of yeast, such as *Pichia* or *Kluyveromyces,* may also be employed. Yeast vectors will often contain an origin of replication sequence from a 2µ yeast plasmid, an autonomously replicating sequence (ARS), a promoter region, sequences for polyadenylation, and sequences for transcription termination. Preferably, yeast vectors include an origin of replication sequence and selectable marker. Suitable promoter sequences for yeast vectors include promoters for metallothionein, 3-phosphoglycerate kinase (Hitzeman et al., *J. Biol. Chem. 255*:2073, 1980) or other glycolytic enzymes (Hess et al., *J. Adv. Enzyme Reg. 7*:149, 1968: and Holland et al., *Biochem. 17*:4900, 1978), such as enolase. glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other suitable vectors and promoters for use in yeast expression are further described in Hitzeman, EPA-73,657.

Yeast vectors can be assembled, for example, using DNA sequences from pBR322 for selection and replication in *E. coli* (Amp^{r} gene and origin of replication). Other yeast DNA sequences that can be included in a yeast expression construct include a glucose-repressible ADH2 promoter and α-factor secretion leader. The ADH2 promoter has been described by Russell et al. (*J. Biol. Chem. 258*:2674, 1982) and Beier et al. (*Nature 300*:724, 1982). The yeast α-factor leader sequence directs secretion of heterologous polypeptides. The α-factor leader sequence is often inserted between the promoter sequence and the structural gene sequence. *See, e.g*., Kurjan et al., *Cell 30*:933, 1982 and Bitter et al., *Proc. Natl. Acad. Sci. USA 81*:5330, 1984. Other leader sequences suitable for facilitating secretion of recombinant polypeptides from yeast hosts are known to those of skill in the art. A leader sequence may be modified near its 3' end to contain one or more restriction sites. This will facilitate fusion of the leader sequence to the structural gene.

Yeast transformation protocols are known to those of skill in the art. One such protocol is described by Hinnen et al., *Proc. Natl. Acad. Sci. USA 75*:1929, 1978. For example, one can select for Trp⁺ transformants in a selective medium, wherein the selective medium consists of 0.67% yeast nitrogen base, 0.5% casamino acids, 2% glucose, 10 µg/ml adenine and 20 µg/ml uracil. Yeast host cells transformed by vectors containing ADH2 promoter sequence may be grown for inducing expression in a "rich" medium. An example of a rich medium is one consisting of 1% yeast extract, 2% peptone, and 1% glucose supplemented with 80 µg/ml adenine and 80 µg/ml uracil. Derepression of the ADH2 promoter occurs when glucose is exhausted from the medium.

Mammalian or insect host cell culture systems could also be employed to express recombinant fusion protein. Examples of suitable mammalian host cell lines include the COS-7 line of monkey kidney cells (ATCC CRL 1651; Gluzman et al., *Cell 23*:175, 1981), L cells, C127 cells, 3T3 cells (ATCC CRL 163), Chinese hamster ovary (CHO) cells, HeLa cells, BHK (ATCC CRL 10) cell lines, and CV-1/EBNA cells (ATCC CRL 10478). The CV-1/EBNA cell line was derived by transfection of the CV-1 cell line with a gene encoding Epstein-Barr virus nuclear antigen-1 (EBNA-1) and constitutively express EBNA-1 driven from human CMV immediate-early enhancer/promoter. An EBNA-1 gene allows for episomal replication of expression vectors that contain the EBV origin of replication.

### Protein Purification

Purified soluble fusion proteins are prepared by culturing suitable host/vector systems to express the recombinant soluble fusion proteins, which are then purified from culture media or cell extracts, using standard methods of protein purification that are optimized for each individual soluble fusion protein.

For example, supernatants from systems which secrete recombinant protein into culture media are clarified, and concentrated using a commercially available protein concentration filter, for example, an Amicon or Millipore Pellicon ultrafiltration unit. Following the concentration step, the concentrate can be applied to a suitable purification matrix. Suitable matrices include those useful in affinity chromatography. For example, a suitable affinity matrix can comprise a cognate protein to which the fusion proteins binds, or lectin or antibody molecule which binds the fusion protein. bound to a suitable support.

Alternatively, an ion exchange resin can be employed, for example, an anion exchange resin comprising a matrix or substrate having pendant diethylaminoethyl (DEAE) groups, or other suitable anion exchangers. The matrices can be acrylamide, agarose, dextran, cellulose or other types commonly employed in protein purification. Alternatively, a cation exchange step can be employed. Suitable cation exchangers include various insoluble matrices comprising sulfopropyl or carboxymethyl groups.

One or more reversed-phase high performance liquid chromatography (RP-HPLC) steps employing hydrophobic RP-HPLC media, e.g., silica gel having pendant methyl or other aliphatic groups, can be employed to further purify a soluble fusion protein. Size exclusion chromatography will also be useful in purifying soluble fusion proteins. Additionally, hydrophobic supports can also be used under low pressure conditions; an exemplary medium is phenyl-sepharose. Some or all of the foregoing purification steps, in various combinations, can also be employed to provide a homogeneous recombinant protein.

### Biological Activity

Biological activity of recombinant, soluble fusion proteins is mediated by binding of the recombinant, soluble fusion protein to a cognate molecule. A cognate molecule is defined as a molecule which binds the recombinant soluble fusion protein in a non-covalent interaction based upon the proper conformation of the recombinant soluble fusion protein and the cognate molecule. For example, for a recombinant soluble fusion protein comprising an extracellular region of a receptor, the cognate molecule comprises a ligand which binds the extracellular region of the receptor. Conversely, for a recombinant soluble fusion protein comprising a ligand, the cognate molecule comprises a receptor (or binding protein) which binds the ligand.

Binding of a recombinant fusion protein to a cognate molecule is a marker for biological activity. Such binding activity may be determined, for example, by competition for binding to the binding domain of the cognate molecule (i.e. competitive binding assays). One configuration of a competitive binding assay for a recombinant soluble fusion protein comprising a ligand uses a radiolabeled, soluble receptor, and intact cells expressing a native form of the ligand. Such an assay is illustrated in Example 4 herein. Similarly, a competitive assay for a recombinant soluble fusion protein comprising a receptor uses a radiolabeled, soluble ligand, and intact cells expressing a native form of the receptor. Instead of intact cells expressing a native form of the cognate molecule, one could substitute purified cognate molecule bound to a solid phase. Competitive binding assays can be performed using standard methodology. Qualitative or semi-quantitative results can be obtained by competitive autoradiographic plate binding assays, or fluorescence activated cell sorting, or Scatchard plots may be utilized to generate quantitative results.

Biological activity may also be measured using bioassays that are known in the art, such as a cell proliferation assay. Exemplary bioassays are described in Example 2 herein. The type of cell proliferation assay used will depend upon the recombinant soluble fusion protein. A bioassay for a recombinant soluble fusion protein that in its native form acts upon T cells will utilize purified T cells obtained by methods that are known in the art. Such bioassays include costimulation assays in which the purified T cells are incubated in the presence of the recombinant soluble fusion protein and a suboptimal level of a mitogen such as Con A or PHA. Similarly, purified B cells will be used for a recombinant soluble fusion protein that in its native form acts upon B cells. Other types of cells may also be selected based upon the cell type upon which the native form of the recombinant soluble fusion protein acts. Proliferation is determined by measuring the incorporation of a radiolabeled substance, such as ³H thymidine, according to standard methods.

Yet another type assay for determining biological activity is induction of secretion of secondary molecules. For example, certain proteins induce secretion of cytokines by T cells. T cells are purified and stimulated with a recombinant soluble fusion protein under the conditions required to induce cytokine secretion (for example, in the presence of a comitogen). Induction of cytokine secretion is determined by bioassay, measuring the proliferation of a cytokine dependent cell line. Similarly, induction of immunoglobulin secretion is determined by measuring the amount of immunoglobulin secreted by purified B cells stimulated with a recombinant soluble fusion protein that acts on B cells in its native form, using a quantitative (or semi-quantitative) assay such as an enzyme immunoassay. Example 2 presents such assays.

The relevant disclosures of all references cited herein are specifically incorporated by reference. The following examples are offered by way of illustration, and not by way of limitation.

### EXAMPLE 1

This example describes construction of a CD40-L DNA construct to express a soluble CD40-L fusion protein referred to as trimeric CD40-L. CD40-L is a type II transmembrane protein found on activated T cells, that acts as a ligand for the B cell antigen, CD40 (Armitage et al., *Nature* 357:80, 1992; Spriggs et al., *J. Exp. Med*. 176:1543, 1992). A gene encoding CD40-L has been cloned and sequenced as described in U.S.S.N. 07/969,703, filed October 23, 1992, the disclosure of which is incorporated by reference herein. CD40-L is a member of the Tumor Necrosis Factor (TNF) family of proteins: several members of this family are believed to exist in trimeric form.

Trimeric CD40-L contains a leader sequence, a 33 amino acid sequence referred to as a "leucine zipper" (SEQ ID NO:2), and an eight amino acid hydrophilic sequence described by Hopp et al. (Hopp et al., *Bio*/*Technology* 6:1204, 1988; SEQ ID NO:1; referred to as Flag®), followed by the extracellular region of human CD40-L (amino acid 50 to amino acid 261 of SEQ ID NOs:3 and 4). The utility of the leader and the Flag® sequences have been described in previously. The 33 amino acid sequence presented in SEQ ID NO:2 trimerizes spontaneously in solution. Fusion proteins comprising this 33 amino acid sequence are thus expected to form trimers or multimers spontaneously.

The construct is prepared by synthesizing oligonucleotides representing a leader sequence, the 33 amino acid sequence described above (SEQ ID NO:2), and the Flag® sequence (SEQ ID NO: 1), then ligating the final product to a DNA fragment encoding the extracellular region of human CD40-L (amino acids 50 to 261 of SEQ ID NOs:3 and 4).

The resulting ligation product in expression vector pDC406 was transfected into the monkey kidney cell line CV-1/EBNA (ATCC CRL 10478). The pDC406 plasmid includes regulatory sequences derived from SV40, human immunodeficiency virus (HIV), and Epstein-Barr virus (EBV). The CV-1/EBNA cell line was derived by transfection of the CV-1 cell line with a gene encoding Epstein-Barr virus nuclear antigen-1 (EBNA-1) that constitutively expresses EBNA-1 driven from the human CMV intermediate-early enhancer/promoter. The EBNA-1 gene allows for episomal replication of expression vectors, such as pDC406, that contain the EBV origin of replication.

Once cells expressing the fusion construct are identified, large scale cultures of transfected cells are grown to accumulate supernatant from cells expressing soluble, oligomeric CD40-L. The soluble, oligomeric CD40-L fusion protein in supernatant fluid is purified by affinity purification substantially as described in U.S. Patent 5,011,912. sCD40-L may also be purified using other protein purification methods, as described herein. Silver-stained SDS gels of the soluble, oligomeric CD40-L fusion protein can be prepared to determine purity. Similar methods are used to prepare and purify a trimer-forming construct comprising the extracellular region of murine CD40-L (amino acid 50 to amino acid 260 of SEQ ID NOs:5 and 6). Soluble CD40-L exhibits similar biological activity to that of membrane-bound CD40-L, as shown in Example 2.

### Example 2

This example illustrates B cell proliferative activity and induction of polyclonal immunoglobulin secretion using soluble, oligomeric CD40-L prepared as described in Example 1. Human B cells were purified substantially as described in Armitage et al. (*J. Immunol*. 150:3671; 1993). Briefly, tonsillar tissue was gently teased and the resulting cell suspension centrifuged over Histopaque® (Sigma, St. Louis, MO). T cell-depleted preparations of cells (E-) were obtained by removing T cells by rosetting with 2-aminoethylisothiouronium bromide-treated SRBC (sheep red blood cells) and treatment with B cell Lympho-kwik (One Lambda Inc., Los Angeles, CA) for 1 hour at 37°C to lyse contaminating non-B cells. Peripheral blood mononuclear cells (PBMC) were isolated in the same manner, with the additional step of treating the partially purified cells with 5 mM leucine methyl ester (Leu ME; Sigma, St. Louis, MO) in serum-free medium for one hour at room temperature prior to the Lympho-kwik step, to remove phagocytic cells.

B cell proliferation was measured with a ³H-thymidine incorporation assay, substantially as described in Armitage et al., *supra.* Cells were cultured for three days in the presence of soluble, oligomeric CD40-L, alone or in the presence of 5 ng/ml IL-4 (Immunex Corporation, Seattle, WA), 5 µg/ml anti-IgM coated beads (BioRad, Richmond, CA), or a combination of IL-4 and anti-IgM. The results of a representative experiment to evaluate the ability of soluble, oligomeric CD40-L to induce B cell proliferation are shown in Figures 1A and 1B. sCD40-L induced proliferation of tonsillar B cells in the presence of IL-4, anti-IgM, or a combination of these to co-factors (Figure 1A). sCD40-L also induced proliferation of peripheral blood B cells in the presence of IL-4, anti-IgM, or a combination of these to co-factors, and with B cells obtained from some donors, a moderate level of proliferation in the absence of any cofactor (Figure 1B). These results parallel the results obtained with recombinant, membrane-bound CD40-L described in Armitage et al. *supra*.

Polyclonal immunoglobulin secretion was determined by isotype-specific ELISA on supernatant fluid from 10 day cultures of 1 X 10⁵ B cells per well, substantially as described in Armitage et al., *supra.* Purified B cells were stimulated with a 1:20 dilution of supernatant fluid containing soluble, oligomeric CD40-L (sCD40-L), a 1:20 dilution of control supernatant (control S/N; conditioned medium from cells transfected with vector alone), or transfected CV-1/EBNA cells expressing membrane-bound CD40-L (CV1/CD40L; 3 x 10⁴ cells/well), in the presence or absence of 10 ng/ml of either IL-2, IL-4 (both from Immunex Corporation, Seattle, WA) or IL-10 (Genzyme Corporation, Boston, MA). The results of a representative experiment measuring immunoglobulin secretion are presented in Table 1; values given represent the quantity of each isotype secreted by the induced B cells in ng/ml, as measured by ELISA.

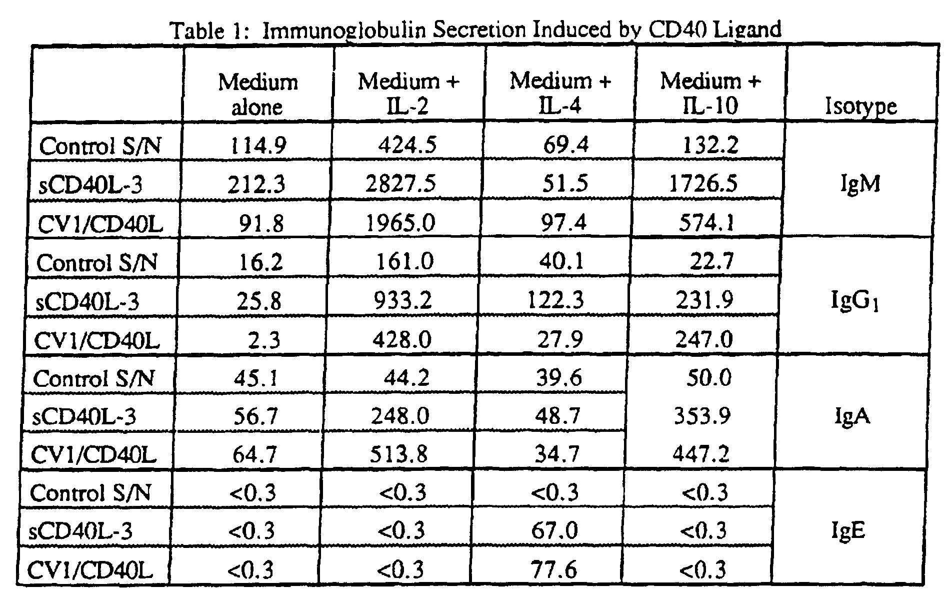

These results indicated that soluble, oligomeric CD40-L induced polyclonal immunoglobulin secretion in the same manner as membrane-bound CD40-L. IL-2 and IL-10 enhanced secretion of IgM, IgG₁ and IgA; secretion of measurable amounts of IgE occurred only in the presence of IL-4, just as observed for membrane-bound CD40-L. The same pattern of immunoglobulin secretion was present when B cells from several different donors were tested, although the absolute quantities varied from donor to donor. In similar experiments in a murine system, a soluble, oligomeric construct of a murine CD40-L also gave comparable results to membrane-bound murine CD40-L.

### Example 3

This example describes construction of a CD27-L DNA construct to express a soluble, oligomeric CD27-L fusion protein referred to as sCD27L-3. CD27-L is a type II transmembrane protein that binds to the lymphocyte antigen, CD27. CD27 is found on most peripheral blood T cells (Bigler et al., *J. Immunol*. 141:21, 1988; van Lier et al., *Eur. J. Immunol*. 18:811, 1988), and a subpopulation of B cells (Maurer et al., *Eur. J. Immunol.* 20:2679. 1990). CD27-L is a member of the tumor necrosis factor family of cytokines. A gene encoding CD27-L has been cloned and sequenced as described in Goodwin et al., *Cell* 73:447 (1993), and in U.S.S.N. , filed , a continuation-in-part of U.S.S.N. 07/941,648, filed September 8, 1992, the disclosures of which are incorporated by reference herein.

The construct encoding sCD27L-3 contains a leader sequence, a 37 amino acid sequence comprising a leucine zipper domain, and the extracellular region of human CD27-L from amino acid 39 to amino acid 193; the nucleotide and amino acids sequences are presented in SEQ ID NOs:7 and 7. The construct was prepared by using methods that are well-known in the art to obtain a DNA encoding the extracellular region of CD27-L. Briefly, the extracellular region of CD27-L was amplified from a full-length CD27-L cDNA using a PCR technique. The primers used were derived from the extracellular region of CD27-L (SEQ ID NO:7, nucleotides 222-245, for the 5' primer , and the complement of nucleotides 663-689 for the 3' primer) with addition of sequences encoding desired restriction enzyme sites (ACTAGT, which contains a *Spe* I site, for the 5' primer, and GCGGCCGC, which contains a *Not* I site, for the 3' primer). The amplified PCR product, representing the extracellular domain of CD27-L, was cloned into an *Spe* I/*Not* I-cut SMAG (pDC206) vector. SMAG vector is a derivative of pDC201 (Sims et al., *Science* 241:585, 1988) that contains the murine IL-7 leader sequence. The vector was amplified, then cut with *Spe* I and treated with calf intestinal alkaline phosphatase. Oligonucleotides based on the amino acid sequence of a leucine zipper (SEQ ID NO:1) were synthesized by standard methodology, and ligated with the *Spe* I-cut vector, to form an expression vector comprising a murine IL-7 leader sequence (Namen et al., *Nature* 333:571; 1988), a leucine zipper domain, and the extracellular domain of CD27-L. The expression vector was referred to as pDC206/sCD27L-3.

pDC206/sCD27L-3 was co-transfected into the monkey kidney cell line CV-1/EBNA (ATCC CRL 10478) along with a pSV3Neo plasmid. pSV3Neo (Mulligan and Berg, *Proc. Natl. Acad. Sci. U.S.A*. 78:2072; 1981) is a plasmid which expresses the SV40 T antigen, and thus allows for the episomal replication of the pDC206 plasmid.

Once cells expressing the fusion construct are identified, large scale cultures of transfected cells are grown to accumulate supernatant from cells expressing the soluble, oligomeric CD27-L fusion protein (referred to as sCD27L-3). sCD27L-3 in supernatant fluid is purified by affinity purification substantially as described in U.S. Patent 5,011,912. sCD27L-3 may also be purified using other protein purification methods, as described herein. Silver-stained SDS gels of the soluble, oligomeric CD27-L fusion protein can be prepared to determine purity. sCD27L-3 binds to soluble CD27, and inhibits binding of soluble CD27 to cells expressing CD27-L, as described in Example 4.

### Example 4

This example illustrates a binding inhibition activity of sCD27L-3. A soluble form of the human lymphocyte surface antigen CD27 was prepared substantially as described by Fanslow et al., *J. Immunol.* 149:65 (1992), to form a dimeric, Fc fusion construct referred to as CD27.Fc (Goodwin et al., *Cell* 73:447; *1993).* CD27.Fc comprises the extracellular region of CD27 and an Fc region from a human IgG₁. sCD27L-3 inhibits binding of CD27.Fc to MP.1 cell, a human, Epstein-Barr virus-transformed B cell line that expresses endogenous CD27-L.

Conditioned supernatant fluid from CV-1/EBNA cells transfected with pDC206/sCD27L-3 was titrated in a 96 well plate. A constant amount of CD27.Fc (1 µg/well) was added to each well, followed by 1-2 x 10⁶ MP.1 cells per well, in binding medium (RPMI-1640 containing 1 % bovine serum albumin, 0.2 % sodium azide and 20 mM HEPES, pH 7.2). The plate was incubated at 37°C for one hour. Cells were washed twice with PBS, then pelleted by centrifugation. ¹²⁵I-mouse anti-human IgG Fc was added to each well at a constant concentration, and the plate incubated for an additional hour at 37°C. The ¹²⁵I-mouse anti-human IgG Fc bound to the CD27.Fc that bound to the MP.1 cells. After the final incubation , cells were harvested over pthalate oil-containing tubes to separate the bound and free ¹²⁵I-mouse anti-human IgG Fc, and the amount of radioactivity quantitated using a gamma counter.

The results of this experiment are presented in Figure 2. sCD27L-3 exhibited a dose-dependent inhibition of the binding of CD27.Fc to MP.1 cells. By comparing the concentration at which the inhibition of binding of CD27.Fc is at 50% to the titration of inhibition by sCD27L-3, it was estimated that the concentration of sCD27L-3 in the conditioned medium was between 18 and 40 µg/ml. In making this comparison, the MW of sCD27L-3 was estimated to be 135 Kd (estimated MW of extracellular region of CD27-L was 45 Kd, multiplied by three for formation of trimer), and the binding of sCD27L-3 to CD27.Fc was assumed to occur at a molar ratio. The Kᵢ was estimated to be 10 times the Kₐ, which was 3 x 10⁻⁷M⁻¹, and the initial concentration was assumed to be 1 x 10⁻⁸ M. The results demonstrated that the initial assumption of a concentration of 1 x 10⁻⁸ M was approximately 10-fold too low, and a 1:3 dilution of the supernatant fluid actually gave an estimated concentration of 1 x 10⁻⁷ M.

## Claims

1. A method of preparing a soluble protein, the method comprising the step of culturing a host cell transformed or transfected with an expression vector comprising DNA encoding a protein, the protein selected from the group consisting of:
(a) a leucine zipper domain and an extracellular region of a heterologous mammalian transmembrane protein which forms an oligomer of at least a trimer;
(b) a leucine zipper domain and a soluble homotrimeric forming domain of a protein of the tumour necrosis factor/nerve growth factor family and their receptors;
(c) a leucine zipper domain and a soluble oligomeric forming domain of a cytokine which forms an oligomer of at least a trimer;
wherein, the step of culturing the host cell is carried out under conditions promoting expression of the protein.

2. The method of claim 1 wherein the leucine zipper domain comprises amino acids 1-33 of SEQ ID NO: 2.

3. The method of claim 1 or 2 wherein the protein of the tumour necrosis factor/nerve growth factor family and receptors is selected from the group consisting of:
(a) TNFR;
(b) TNFα;
(c) TNFβ;
(d) CD40L;
(e) CD27L;
(f) OX40L; and
(g) NGFR.

4. The method of claim 1 or 2, wherein the cytokine is GM-CSF.

5. The method of claim 4, wherein the protein further comprises IL-3.

6. A method of preparing a protein, the method comprising the step of culturing a host cell transformed or transfected with an expression vector comprising DNA encoding amino acids 1-33 of SEQ ID NO: 2 and a soluble region of CD40L, wherein the step of culturing the host cell is carried out under conditions promoting expression of the protein.

7. The method of claim 1, 2 or 3, wherein when the heterologous mammalian transmembrane protein is a Type II transmembrane protein, the leucine zipper domain is fused to the amino-proximal end of the extracellular region.

8. The method of claim 1, 2 or 3, wherein when the heterologous mammalian transmembrane protein is a Type I transmembrane protein, the leucine zipper domain is fused to the carboxy-proximal end of the extracellular region.

9. The method according to any one of claims 1 to 8, wherein the protein comprises an extracellular region of a first heterologous mammalian protein and an extracellular region of a second heterologous mammalian protein, and wherein the first and second heterologous mammalian proteins are different.

10. The method according to any of claims 1 to 9, wherein the protein further comprises a linker sequence inserted between the leucine zipper domain and the extracellular region of the transmembrane protein.

11. A protein selected from the group consisting of:
(a) a fusion of a leucine zipper domain and an extracellular region of a heterologous mammalian transmembrane protein which forms an oligomer of at least a trimer;
(b) a fusion of a leucine zipper domain and a soluble homotrimeric forming domain of a protein of the tumour necrosis factor/nerve growth factor family and their receptors; and
(c) a fusion of a leucine zipper domain and a soluble oligomeric forming domain of a cytokine which forms an oligomer of at least a trimer
wherein the leucine zipper domain of each fusion protein is responsible for oligomerisation of the protein.

12. The protein of claim 11 wherein the protein of the tumour necrosis factor/nerve growth factor family and receptors is selected from the group consisting of:
(a) TNFR;
(b) TNFα;
(c) TNFβ;
(d) CD40L;
(e) CD27L;
(f) OX40L; and
(g) NGFR.

13. The protein of claim 11 or 12, wherein the leucine zipper domain comprises amino acids 1-33 of SEQ ID NO: 2.

14. The protein of claim 11 or 12, wherein the cytokine is GM-CSF.

15. The protein of claim 14, wherein the protein further comprises IL-3.

16. The protein of claim11, 12 or 13, wherein when the heterologous mammalian transmembrane protein is a Type II transmembrane protein, the leucine zipper domain is fused to the amino-proximal end of the extracellular region.

17. The protein of claim 11, 12 or 13, wherein when the heterologous mammalian transmembrane protein is a Type I transmembrane protein, the leucine zipper domain is fused to the carboxy-proximal end of the extracellular region.

18. The protein of any of claims 11 -17, further comprising a linker sequence.

19. The protein of claim 18 wherein the linker sequence is a peptide.

20. A protein of any of claims 11-19 wherein the leucine zipper comprises amino acids 1-33 of SEQ ID NO: 2, except for conservative amino acid substitutions.

21. A protein of any of claims 11-20:
(a) amino acids 1-33 of SEQ ID NO: 2, and
(b) an extracellular region of a heterologous mammalian transmembrane protein.

22. The protein of claim 21 wherein the extracellular region is soluble CD40L.

23. A DNA encoding a protein of any of claims 11-22.

## Patentansprüche

1. Verfahren zur Herstellung eines löslichen Proteins, wobei das Verfahren den Schritt des Kultivierens einer Wirtszelle aufweist, die mit einem Expressionsvektor transformiert oder transfektiert ist, der eine DNA enthält, die ein Protein codiert, wobei das Protein aus der Gruppe ausgewählt wird, die besteht aus:
(a) einer Leucin-Zipper-Domäne und einer extrazellulären Region eines heterologen Säugetier-Transmembranproteins, das ein Oligomer von mindestens einem Trimer bildet;
(b) einer Leucin-Zipper-Domäne und einer löslichen Homotrimer bildenden Domäne eines Proteins der Tumomekrosefaktor-/Nervenwachstumsfaktor-Familie und deren Rezeptoren;
(c) einer Leucin-Zipper-Domäne und einer löslichen Oligomer bildenden Domäne eines Cytokins, das ein Oligomer von mindestens einem Trimer bildet,
wobei der Schritt des Kultivierens der Wirtszelle unter Bedingungen ausgeführt wird, welche die Expression des Proteins fördern.

2. Verfahren nach Anspruch 1, wobei die Leucin-Zipper-Domäne Aminosäuren 1-33 der SEQ-ID Nr. 2 enthält.

3. Verfahren nach Anspruch 1 oder 2, wobei das Protein der Tumornekrosefaktor-/Nervenwachstumsfaktor-Familie und Rezeptoren aus der Gruppe ausgewählt wird, die besteht aus:
(a) TNFR,
(b) TNFα,
(c) TNFβ,
(d) CD40L,
(e) CD27L,
(f) OX40L und
(g) NGFR.

4. Verfahren nach Anspruch 1 oder 2, wobei das Cytokin GM-CSF ist.

5. Verfahren nach Anspruch 4, wobei das Protein ferner IL-3 umfasst.

6. Verfahren zur Herstellung eines Proteins, wobei das Verfahren den Schritt des Kultivierens einer Wirtszelle aufweist, die mit einem Expressionsvektor transformiert oder transfektiert wird, welcher eine DNA, die Aminosäuren 1-33 der SEQ-ID Nr. 2 codiert, und eine lösliche Region von CD40L enthält, wobei der Schritt des Kultivierens der Wirtszelle unter Bedingungen ausgeführt wird, welche die Expression des Proteins fördern.

7. Verfahren nach Anspruch 1, 2 oder 3, wobei für den Fall, dass das heterologe Säugetier-Transmembranprotein ein Typ-II-Transmembranprotein ist, die Leucin-Zipper-Domäne mit dem amino-proximalen Ende der extrazellulären Region verschmolzen wird.

8. Verfahren nach Anspruch 1, 2 oder 3, wobei für den Fall, dass das heterologe Säugetier-Transmembranprotein ein Typ-I-Transmembranprotein ist, die Leucin-Zipper-Domäne mit dem carboxy-proximalen Ende der extrazellulären Region verschmolzen wird.

9. Verfahren nach einem der Ansprüche 1 bis 8, wobei das Protein eine extrazelluläre Region eines ersten heterologen Säugetier-Proteins und eine extrazelluläre Region eines zweiten heterologen Säugetier-Proteins enthält und wobei sich das erste und das zweite heterologe Säugetier-Protein voneinander unterscheiden.

10. Verfahren nach einem der Ansprüche 1 bis 9, wobei das Protein ferner eine Linkersequenz aufweist, die zwischen der Leucin-Zipper-Domäne und der extrazellulären Region des Transmembranproteins eingefügt wird.

11. Protein, das aus der Gruppe ausgewählt wird, die besteht aus:
(a) einer Fusion einer Leucin-Zipper-Domäne und einer extrazellulären Region eines heterologen Säugetier-Transmembranproteins, das ein Oligomer von mindestens einem Trimer bildet;
(b) einer Fusion einer Leucin-Zipper-Domäne und einer löslichen Homotrimer bildenden Domäne eines Proteins der Tumomekrosefaktor-/Nervenwachstumsfaktor-Familie und deren Rezeptoren; und
(c) einer Fusion einer Leucin-Zipper-Domäne und einer löslichen Oligomer bildenden Domäne eines Cytokins, das ein Oligomer von mindestens einem Trimer bildet,
wobei die Leucin-Zipper-Domäne jedes Fusionsproteins für die Oligomerisierung des Proteins verantwortlich ist.

12. Protein nach Anspruch 11, wobei das Protein der Tumornekrosefaktor/Nervenwachstumsfaktor-Familie und Rezeptoren aus der Gruppe ausgewählt wird, die besteht aus:
(a) TNFR,
(b) TNFα,
(c) TNFβ,
(d) CD40L,
(e) CD27L,
(f) OX40L und
(g) NGFR.

13. Protein nach Anspruch 11 oder 12, wobei die Leucin-Zipper-Domäne Aminosäuren 1-33 der SEQ-ID. Nr. 2 enthält.

14. Protein nach Anspruch 11 oder 12, wobei das Cytokin GM-CSF ist.

15. Protein nach Anspruch 14, wobei das Protein ferner IL-3 umfasst.

16. Protein nach Anspruch 11, 12 oder 13, wobei für den Fall, dass das heterologe Säugetier-Transmembranprotein ein Typ-II-Transmembranprotein ist, die Leucin-Zipper-Domäne mit dem amino-proximalen Ende der extrazellulären Region verschmolzen ist.

17. Protein nach Anspruch 11, 12 oder 13, wobei für den Fall, dass das heterologe Säugetier-Transmembranprotein ein Typ-I-Transmembranprotein ist, die Leucin-Zipper-Domäne mit dem carboxy-proximalen Ende der extrazellulären Region verschmolzen ist.

18. Protein nach einem der Ansprüche 11 bis 17, das ferner eine Linkersequenz aufweist.

19. Protein nach Anspruch 18, wobei die Linkersequenz ein Peptid ist.

20. Protein nach einem der Ansprüche 11 bis 19, wobei die Leucin-Zipper-Domäne Aminosäuren 1-33 der SEQ-ID. Nr. 2 enthält, mit Ausnahme von konservativen Aminosäureaustauschen.

21. Protein nach einem der Ansprüche 11 bis 20:
(a) Aminosäuren 1-33 der SEQ-ID Nr. 2 und
(b) eine extrazelluläre Region eines heterologen Säugetier-Transmembranproteins.

22. Protein nach Anspruch 21, wobei die extrazelluläre Region lösliches CD40L ist.

23. DNA, die ein Protein nach einem der Ansprüche 11 bis 22 codiert.

## Revendications

1. Procédé de préparation d'une protéine soluble, le procédé comprenant l'étape de mise en culture d'une cellule hôte transformée ou transfectée avec un vecteur d'expression comprenant un ADN codant pour une protéine, la protéine étant sélectionnée parmi le groupe comprenant :
(a) un domaine de glissière à leucine et une région extracellulaire d'une protéine transmembranaire hétérologue de mammifère qui forme un oligomère, au moins un trimère;
(b) un domaine de glissière à leucine et un domaine formant un homotrimère soluble d'une protéine de la famille du facteur de nécrose des tumeurs/facteur de croissance des nerfs et leurs récepteurs;
(c) un domaine de glissière à leucine et un domaine formant un oligomère soluble d'une cytokine qui forme un oligomère, au moins un trimère;
dans lequel l'étape de mise en culture de la cellule hôte est réalisée dans des conditions promouvant une expression de la protéine.

2. Procédé suivant la revendication 1, dans lequel le domaine de glissière à leucine comprend les acides aminés 1-33 de la SEQ ID n° 2.

3. Procédé suivant la revendication 1 ou 2, dans lequel la protéine de la famille du facteur de nécrose des tumeurs/facteur de croissance des nerfs et leurs récepteurs est sélectionnée parmi le groupe comprenant :
(a) le TNFR;
(b) le TNFα;
(c) le TNFβ;
(d) le CD40L;
(e) le CD27L;
(f) l'OX40L; et
(g) le NGFR.

4. Procédé suivant la revendication 1 ou 2, dans lequel la cytokine et le GM-CSF.

5. Procédé suivant la revendication 4, dans lequel la protéine comprend en outre l'IL-3.

6. Procédé de préparation d'une protéine, le procédé comprenant l'étape de la mise en culture d'une cellule hôte transformée ou transfectée avec un vecteur d'expression comprenant de l'ADN codant pour les acides aminés 1-33 de la SEQ ID n° 2 et une région soluble du CD40L, dans lequel l'étape de mise en culture de la cellule hôte est réalisée dans des conditions promouvant une expression de la protéine.

7. Procédé suivant la revendication 1, 2 ou 3, dans lequel lorsque la protéine transmembranaire hétérologue de mammifère est une protéine transmembranaire de type II, le domaine de glissière à leucine est fusionné à l'extrémité du côté amino de la région extracellulaire.

8. Procédé suivant la revendication 1, 2 ou 3, dans lequel lorsque la protéine transmembranaire hétérologue de mammifère est une protéine transmembranaire de type I, le domaine de glissière à leucine est fusionné à l'extrémité du côté carboxy de la région extracellulaire.

9. Procédé suivant l'une quelconque des revendications 1 à 8, dans lequel la protéine comprend une région extracellulaire d'une première protéine hétérologue de mammifère et une région extracellulaire d'une deuxième protéine hétérologue de mammifère, et dans lequel les première et deuxième protéines hétérologues de mammifère sont différentes.

10. Procédé suivant l'une quelconque des revendications 1 à 9, dans lequel la protéine comprend en outre une séquence de bras de liaison insérée entre le domaine de glissière à leucine et la région extracellulaire de la protéine transmembranaire.

11. Protéine sélectionnée parmi le groupe comprenant :
(a) une fusion d'un domaine de glissière à leucine et d'une région extracellulaire d'une protéine transmembranaire hétérologue de mammifère qui forme un oligomère, au moins un trimère;
(b) une fusion d'un domaine de glissière à leucine et d'un domaine formant un homotrimère soluble d'une protéine de la famille du facteur de nécrose des tumeurs/facteur de croissance des nerfs et leurs récepteurs; et
(c) une fusion d'un domaine de glissière à leucine et d'un domaine formant un oligomère soluble d'une cytokine qui forme un oligomère, au moins un trimère;
dans lequel le domaine de glissière à leucine de chaque protéine de fusion est responsable de la formation d'oligomère par la protéine.

12. Protéine suivant la revendication 11, dans laquelle la protéine de la famille du facteur de nécrose des tumeurs/facteur de croissance des nerfs et leurs récepteurs est sélectionnée parmi le groupe comprenant :
(a) le TNFR;
(b) le TNFα;
(c) le TNFβ;
(d) le CD40L;
(e) le CD27L;
(f) l'OX40L; et
(g) le NGFR.

13. Protéine suivant la revendication 11 ou 12, dans laquelle le domaine de glissière à leucine comprend les acides aminés 1-33 de la SEQ ID n° 2.

14. Protéine suivant la revendication 11 ou 12, dans laquelle la cytokine et le GM-CSF.

15. Protéine suivant la revendication 14, dans laquelle la protéine comprend en outre l'IL-3.

16. Protéine suivant la revendication 11, 12 ou 13, dans laquelle lorsque la protéine transmembranaire hétérologue de mammifère est une protéine transmembranaire de type II, le domaine de glissière à leucine est fusionné à l'extrémité du côté amino de la région extracellulaire.

17. Protéine suivant la revendication 11, 12 ou 13, dans laquelle lorsque la protéine transmembranaire hétérologue de mammifère est une protéine transmembranaire de type I, le domaine de glissière à leucine est fusionné à l'extrémité du côté carboxy de la région extracellulaire.

18. Protéine suivant l'une quelconque des revendications 11 à 17, comprenant en outre une séquence de bras de liaison.

19. Protéine suivant la revendication 18, dans laquelle la séquence de bras de liaison est un peptide.

20. Protéine suivant l'une quelconque des revendications 11-19, dans laquelle la glissière à leucine comprend les acides aminés 1-33 de la SEQ ID n° 2, à l'exception de substitutions conservatrices d'acides aminés.

21. Protéine suivant l'une quelconque des revendications 11-20 :
(a) les acides aminés 1-33 de la SEQ ID n° 2,
(b) une région extracellulaire d'une protéine transmembranaire hétérologue de mammifère.

22. Protéine suivant la revendication 21, dans laquelle la région extracellulaire est le CD40L soluble.

23. ADN codant pour une protéine suivant l'une quelconque des revendications 11-22.


### SEQUENCE LISTING

(1) GENERAL INFORMATION:
   (i) APPLICANT:
      Spriggs, Melanie
      Srinivasan, Subhashini
   (ii) TITLE OF INVENTION: Methods of Preparing Soluble, Oligomeric Proteins
   (iii) NUMBER OF SEQUENCES: 8
   (iv) CORRESPONDENCE ADDRESS:
      (A) ADDRESSEE: Immunex Corporation
      (B) STREET: 51 University Street
      (C) CITY: Seattle
      (D) STATE: WA
      (E) COUNTRY: USA
      (F) ZIP: 98101
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Perkins, Patricia A
      (B) REGISTRATION NUMBER: 34,693
      (C) REFERENCE/DOCKET NUMBER: 1003
   (ix) TELECOMMUNICATION INFORMATION:
      (A) TELEPHONE: (206)587-0430
      (B) TELEFAX: (206)233-0644
(2) INFORMATION FOR SEQ ID NO:1:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (xi) SEQUENCE DESCRIPTION: SEQ ID NO:1:
   Asp Tyr Lys Asp Asp Asp Asp Lys
   1               5
(2) INFORMATION FOR SEQ ID NO:2:
   (i) SEQUENCE CHARACTERISTICS:
      (A) LENGTH: 33 amino acids
      (B) TYPE: amino acid
      (D) TOPOLOGY: linear
   (ii) MOLECULE TYPE: peptide
   (xi) SEQUENCE DESCRIPTION: SEQ ID NO:2:
   Arg Met Lys Gln Ile Glu Asp Lys Ile Glu Glu Ile Leu Ser Lys Ile
   1               5                   10                  15
   Tyr His Ile Glu Asn Glu Ile Ala Arg Ile Lys Lys Leu Ile Gly Glu
               20                  25                  30
   Arg
(2) INFORMATION FOR SEQ ID NO:3:
   (i) SEQUENCE CHARACTERISTICS:
      (A) LENGTH: 786 base pairs
      (B) TYPE: nucleic acid
      (C) STRANDEDNESS: single
      (D) TOPOLOGY: linear
   (ii) MOLECULE TYPE: cDNA to mRNA
   (iii) HYPOTHETICAL: NO
   (iv) ANTI-SENSE: NO
   (vi) ORIGINAL SOURCE:
      (A) ORGANISM: Human
      (B) STRAIN: CD40-L
   (ix) FEATURE:
      (A) NAME/KEY: CDS
      (B) LOCATION: 1..783
   (xi) SEQUENCE DESCRIPTION: SEQ ID NO:3:
ATG ATC GAA ACA TAC AAC CAA ACT TCT CCC CGA TCT GCG GCC ACT GGA    48
   CTG CCC ATC AGC ATG AAA ATT TTT ATG TAT TTA CTT ACT GTT TTT CTT    96
   ATC ACC CAG ATG ATT GGG TCA GCA CTT TTT GCT GTG TAT CTT CAT AGA   144
   AGG TTG GAC AAG ATA GAA GAT GAA AGG AAT CTT CAT GAA GAT TTT GTA   192
   TTC ATG AAA ACG ATA CAG AGA TGC AAC ACA GGA GAA AGA TCC TTA TCC   240
   TTA CTG AAC TGT GAG GAG ATT AAA AGC CAG TTT GAA GGC TTT GTG AAG   288
   GAT ATA ATG TTA AAC AAA GAG GAG ACG AAG AAA GAA AAC AGC TTT GAA   336
   ATG CAA AAA GGT GAT CAG AAT CCT CAA ATT GCG GCA CAT GTC ATA AGT   384
   GAG GCC AGC AGT AAA ACA ACA TCT GTG TTA CAG TGG GCT GAA AAA GGA   432
   TAC TAC ACC ATG AGC AAC AAC TTG GTA ACC CTG GAA AAT GGG AAA CAG   480
   CTG ACC GTT AAA AGA CAA GGA CTC TAT TAT ATC TAT GCC CAA GTC ACC   528
   TTC TGT TCC AAT CGG GAA GCT TCG AGT CAA GCT CCA TTT ATA GCC AGC   576
   CTC TGC CTA AAG TCC CCC GGT AGA TTC GAG AGA ATC TTA CTC AGA GCT   624
   GCA AAT ACC CAC AGT TCC GCC AAA CCT TGC GGG CAA CAA TCC ATT CAC   672
   TTG GGA GGA GTA TTT GAA TTG CAA CCA GGT GCT TCG GTG TTT GTC AAT   720
   GTG ACT GAT CCA AGC CAA GTG AGC CAT GGC ACT GGC TTC ACG TCC TTT   768
   GGC TTA CTC AAA CTC TGA                                           786
(2) INFORMATION FOR SEQ ID NO:4:
   (i) SEQUENCE CHARACTERISTICS:
      (A) LENGTH: 261 amino acids
      (B) TYPE: amino acid
      (D) TOPOLOGY: linear
   (ii) MOLECULE TYPE: protein
   (xi) SEQUENCE DESCRIPTION: SEQ ID NO:4:
(2) INFORMATION FOR SEQ ID NO:5:
   (i) SEQUENCE CHARACTERISTICS:
      (A) LENGTH: 783 base pairs
      (B) TYPE: nucleic acid
      (C) STRANDEDNESS: single
      (D) TOPOLOGY: linear
   (ii) MOLECULE TYPE: cDNA to mRNA
   (iii) HYPOTHETICAL: NO
   (iv) ANTI-SENSE: NO
   (vi) ORIGINAL SOURCE:
      (A) ORGANISM: Mouse
      (B) STRAIN: CD40-L
   (ix) FEATURE:
      (A) NAME/KEY: CDS
      (B) LOCATION: 1..780
   (xi) SEQUENCE DESCRIPTION: SEQ ID NO:5:
   ATG ATA GAA ACA TAC AGC CAA CCT TCC CCC AGA TCC GTG GCA ACT GGA    48
   Met Ile Glu Thr Tyr Ser Gln Pro Ser Pro Arg Ser Val Ala Thr Gly
    1               5                  10                  15
   CTT CCA GCG AGC ATG AAG ATT TTT ATG TAT TTA CTT ACT GTT TTC CTT    96
   Leu Pro Ala Ser Met Lys Ile Phe Met Tyr Leu Leu Thr Val Phe Leu
               20                  25                  30
   ATC ACC CAA ATG ATT GGA TCT GTG CTT TTT GCT GTG TAT CTT CAT AGA   144
   Ile Thr Gln Met Ile Gly Ser Val Leu Phe Ala Val Tyr Leu His Arg
           35                  40                  45
   AGA TTG GAT AAG GTC GAA GAG GAA GTA AAC CTT CAT GAA GAT TTT GTA   192
   Arg Leu Asp Lys Val Glu Glu Glu Val Asn Leu His Glu Asp Phe Val
       50                  55                  60
   TTC ATA AAA AAG CTA AAG AGA TGC AAC AAA GGA GAA GGA TCT TTA TCC   240
   Phe Ile Lys Lys Leu Lys Arg Cys Asn Lys Gly Glu Gly Ser Leu Ser
   65                  70                  75                  80
   TTG CTG AAC TGT GAG GAG ATG AGA AGG CAA TTT GAA GAC CTT GTC AAG   288
   Leu Leu Asn Cys Glu Glu Met Arg Arg Gln Phe Glu Asp Leu Val Lys
                   85                  90                  95
   GAT ATA ACG TTA AAC AAA GAA GAG AAA AAA GAA AAC AGC TTT GAA ATG   336
   Asp Ile Thr Leu Asn Lys Glu Glu Lys Lys Glu Asn Ser Phe Glu Met
               100                 105                 110
   CAA AGA GGT GAT GAG GAT CCT CAA ATT GCA GCA CAC GTT GTA AGC GAA   384
   Gln Arg Gly Asp Glu Asp Pro Gln Ile Ala Ala His Val Val Ser Glu
           115                 120                 125
   GCC AAC AGT AAT GCA GCA TCC GTT CTA CAG TGG GCC AAG AAA GGA TAT   432
   Ala Asn Ser Asn Ala Ala Ser Val Leu Gln Trp Ala Lys Lys Gly Tyr
       130                 135                 140
   TAT ACC ATG AAA AGC AAC TTG GTA ATG CTT GAA AAT GGG AAA CAG CTG   480
   Tyr Thr Met Lys Ser Asn Leu Val Met Leu Glu Asn Gly Lys Gln Leu
   145                 150                 155                 160
   ACG GTT AAA AGA GAA GGA CTC TAT TAT GTC TAC ACT CAA GTC ACC TTC   528
   Thr Val Lys Arg Glu Gly Leu Tyr Tyr Val Tyr Thr Gln Val Thr Phe
                   165                 170                 175
   TGC TCT AAT CGG GAG CCT TCG AGT CAA CGC CCA TTC ATC GTC GGC CTC   576
   Cys Ser Asn Arg Glu Pro Ser Ser Gln Arg Pro Phe Ile Val Gly Leu
               180                 185                 190
   TGG CTG AAG CCC AGC AGT GGA TCT GAG AGA ATC TTA CTC AAG GCG GCA   624
   Trp Leu Lys Pro Ser Ser Gly Ser Glu Arg Ile Leu Leu Lys Ala Ala
           195                 200                 205
   AAT ACC CAC AGT TCC TCC CAG CTT TGC GAG CAG CAG TCT GTT CAC TTG   672
   Asn Thr His Ser Ser Ser Gln Leu Cys Glu Gln Gln Ser Val His Leu
       210                 215                 220
   GGC GGA GTG TTT GAA TTA CAA GCT GGT GCT TCT GTG TTT GTC AAC GTG   720
   Gly Gly Val Phe Glu Leu Gln Ala Gly Ala Ser Val Phe Val Asn Val
   225                 230                 235                 240
   ACT GAA GCA AGC CAA GTG ATC CAC AGA GTT GGC TTC TCA TCT TTT GGC   768
   Thr Glu Ala Ser Gln Val Ile His Arg Val Gly Phe Ser Ser Phe Gly
                   245                 250                 255
   TTA CTC AAA CTC TGA                                               783
   Leu Leu Lys Leu
               260
(2) INFORMATION FOR SEQ ID NO:6:
   (i) SEQUENCE CHARACTERISTICS:
      (A) LENGTH: 260 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Met Ile Glu Thr Tyr Ser Gln Pro Ser Pro Arg Ser Val Ala Thr Gly
     1               5                  10                  15
   Leu Pro Ala Ser Met Lys Ile Phe Met Tyr Leu Leu Thr Val Phe Leu
               20                  25                  30
   Ile Thr Gln Met Ile Gly Ser Val Leu Phe Ala Val Tyr Leu His Arg
           35                  40                  45
   Arg Leu Asp Lys Val Glu Glu Glu Val Asn Leu His Glu Asp Phe Val
       50                  55                  60
   Phe Ile Lys Lys Leu Lys Arg Cys Asn Lys Gly Glu Gly Ser Leu Ser
    65                  70                  75                  80
   Leu Leu Asn Cys Glu Glu Met Arg Arg Gln Phe Glu Asp Leu Val Lys
                    85                  90                  95
   Asp Ile Thr Leu Asn Lys Glu Glu Lys Lys Glu Asn Ser Phe Glu Met
               100                 105                 110
   Gln Arg Gly Asp Glu Asp Pro Gln Ile Ala Ala His Val Val Ser Glu
           115                 120                 125
   Ala Asn Ser Asn Ala Ala Ser Val Leu Gln Trp Ala Lys Lys Gly Tyr
       130                 135                 140
   Tyr Thr Met Lys Ser Asn Leu Val Met Leu Glu Asn Gly Lys Gln Leu
   145                 150                 155                 160
   Thr Val Lys Arg Glu Gly Leu Tyr Tyr Val Tyr Thr Gln Val Thr Phe
                   165                 170                 175
   Cys Ser Asn Arg Glu Pro Ser Ser Gln Arg Pro Phe Ile Val Gly Leu
               180                 185                 190
   Trp Leu Lys Pro Ser Ser Gly Ser Glu Arg Ile Leu Leu Lys Ala Ala
           195                 200                 205
   Asn Thr His Ser Ser Ser Gln Leu Cys Glu Gln Gln Ser Val His Leu
       210                 215                 220
   Gly Gly Val Phe Glu Leu Gln Ala Gly Ala Ser Val Phe Val Asn Val
   225                 230                 235                 240
   Thr Glu Ala Ser Gln Val Ile His Arg Val Gly Phe Ser Ser Phe Gly
                   245                 250                 255
   Leu Leu Lys Leu
               260
(2) INFORMATION FOR SEQ ID NO:7:
   (i) SEQUENCE CHARACTERISTICS:
      (A) LENGTH: 689 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: CD27 ligand trimer (CD27L-3)
   (ix) FEATURE:
      (A) NAME/KEY: CDS
      (B) LOCATION: 39..686
   (ix) FEATURE:
      (A) NAME/KEY: sig_peptide
      (B) LOCATION: 39..110
   (ix) FEATURE:
      (A) NAME/KEY: mat_peptide
      (B) LOCATION: 111..686
   (xi) SEQUENCE DESCRIPTION: SEQ ID NO:7:
   GGAAAACTCT CGAGGTACCT ATCCCGGGGA TCCCCACC ATG TTC CAT GTC TCT      53
                                             Met Phe His Val Ser
                                             -24             -20
   TTT AGA TAT ATC TTT GGA ATT CCT CCA CTG ATC CTT GTT CTG CTG CCT   101
   Phe Arg Tyr Ile Phe Gly Ile Pro Pro Leu Ile Leu Val Leu Leu Pro
                   -15                 -10                  -5
   GTC ACT AGT TCT GAC CGT ATG AAA CAG ATA GAG GAT AAG ATC GAA GAG   149
   Val Thr Ser Ser Asp Arg Met Lys Gln Ile Glu Asp Lys Ile Glu Glu
                 1               5                  10
   ATC CTA AGT AAG ATT TAT CAT ATA GAG AAT GAA ATC GCC CGT ATC AAA   197
   Ile Leu Ser Lys Ile Tyr His Ile Glu Asn Glu Ile Ala Arg Ile Lys
        15                  20                  25
   AAG CTG ATT GGC GAG CGG ACT AGT CAG CGC TTC GCA CAG GCT CAG CAG   245
   Lys Leu Ile Gly Glu Arg Thr Ser Gln Arg Phe Ala Gln Ala Gln Gln
    30                  35                  40                  45
   CAG CTG CCG CTC GAG TCA CTT GGG TGG GAC GTA GCT GAG CTG CAG CTG   293
   Gln Leu Pro Leu Glu Ser Leu Gly Trp Asp Val Ala Glu Leu Gln Leu
                    50                  55                  60
   AAT CAC ACA GGA CCT CAG CAG GAC CCC AGG CTA TAC TGG CAG GGG GGC   341
   Asn His Thr Gly Pro Gln Gln Asp Pro Arg Leu Tyr Trp Gln Gly Gly
                65                  70                  75
   CCA GCA CTG GGC CGC TCC TTC CTG CAT GGA CCA GAG CTG GAC AAG GGG   389
   Pro Ala Leu Gly Arg Ser Phe Leu His Gly Pro Glu Leu Asp Lys Gly
            80                  85                  90
   CAG CTA CGT ATC CAT CGT GAT GGC ATC TAC ATG GTA CAC ATC CAG GTG   437
   Gln Leu Arg Ile His Arg Asp Gly Ile Tyr Met Val His Ile Gln Val
        95                 100                 105
   ACG CTG GCC ATC TGC TCC TCC ACG ACG GCC TCC AGG CAC CAC CCC ACC   485
   Thr Leu Ala Ile Cys Ser Ser Thr Thr Ala Ser Arg His His Pro Thr
   110                 115                 120                 125
   ACC CTG GCC GTG GGA ATC TGC TCT CCC GCC TCC CGT AGC ATC AGC CTG   533
   Thr Leu Ala Val Gly Ile Cys Ser Pro Ala Ser Arg Ser Ile Ser Leu
                   130                 135                 140
   CTG CGT CTC AGC TTC CAC CAA GGT TGT ACC ATT GTC TCC CAG CGC CTG   581
   Leu Arg Leu Ser Phe His Gln Gly Cys Thr Ile Val Ser Gln Arg Leu
               145                 150                 155
   ACG CCC CTG GCC CGA GGG GAC ACA CTC TGC ACC AAC CTC ACT GGG ACA   629
   Thr Pro Leu Ala Arg Gly Asp Thr Leu Cys Thr Asn Leu Thr Gly Thr
           160                 165                 170
   CTT TTG CCT TCC CGA AAC ACT GAT GAG ACC TTC TTT GGA GTG CAG TGG   677
   Leu Leu Pro Ser Arg Asn Thr Asp Glu Thr Phe Phe Gly Val Gln Trp
       175                 180                 185
   GTG CGC CCC TGA                                                   689
   Val Arg Pro
   190
(2) INFORMATION FOR SEQ ID NO:8:
   (i) SEQUENCE CHARACTERISTICS:
      (A) LENGTH: 216 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Phe His Val Ser Phe Arg Tyr Ile Phe Gly Ile Pro Pro Leu Ile
   -24             -20                 -15                 -10
   Leu Val Leu Leu Pro Val Thr Ser Ser Asp Arg Met Lys Gln Ile Glu
               -5                   1               5
   Asp Lys Ile Glu Glu Ile Leu Ser Lys Ile Tyr His Ile Glu Asn Glu
       10                  15                  20
   Ile Ala Arg Ile Lys Lys Leu Ile Gly Glu Arg Thr Ser Gln Arg Phe
   25                  30                  35                  40
   Ala Gln Ala Gln Gln Gln Leu Pro Leu Glu Ser Leu Gly Trp Asp Val
                   45                  50                  55
   Ala Glu Leu Gln Leu Asn His Thr Gly Pro Gln Gln Asp Pro Arg Leu
               60                  65                  70
   Tyr Trp Gln Gly Gly Pro Ala Leu Gly Arg Ser Phe Leu His Gly Pro
           75                  80                  85
   Glu Leu Asp Lys Gly Gln Leu Arg Ile His Arg Asp Gly Ile Tyr Met
       90                  95                  100
   Val His Ile Gln Val Thr Leu Ala Ile Cys Ser Ser Thr Thr Ala Ser
   105                 110                 115                 120
   Arg His His Pro Thr Thr Leu Ala Val Gly Ile Cys Ser Pro Ala Ser
                   125                 130                 135
   Arg Ser Ile Ser Leu Leu Arg Leu Ser Phe His Gln Gly Cys Thr Ile
               140                 145                 150
   Val Ser Gln Arg Leu Thr Pro Leu Ala Arg Gly Asp Thr Leu Cys Thr
           155                 160                 165
   Asn Leu Thr Gly Thr Leu Leu Pro Ser Arg Asn Thr Asp Glu Thr Phe
       170                 175                 180
   Phe Gly Val Gln Trp Val Arg Pro
   185                 190